# FASCICULE DE BREVET EUROPEEN

(11) **EP 1 202 766 B1**
(45) Date de publication et mention de la délivrance du brevet: **15.09.2004**
(21) Numéro de dépôt: 00949593.8
(22) Date de dépôt: 30.06.2000
(51) Int. Cl.: A61M 5/30

(54) **SERINGUE SANS AIGUILLE AVEC INJECTEUR A ELEMENTS EMBOITES**
NADELLOSE SPRITZE MIT INEINANDERGEFÜGTEN INJEKTORELEMENTEN
NEEDLELESS SYRINGE COMPRISING AN INJECTOR WITH NESTED ELEMENTS

(30) Priorité: 16.07.1999 FR 9909252
(43) Date de publication de la demande: 08.05.2002
(73) Titulaire: CROSSJECT, 75181 Paris Cedex 04 (FR)
(72) Inventeur: ALEXANDRE, Patrick, F-70100 Gray (FR); BROUQUIERES, Bernard, F-83100 Toulon (FR); MIKLER, Claude, F-21000 Dijon (FR); NAVELIER, Alain, F-83390-Pierrefeu du Var (FR)
(74) Mandataire: Waligorski, Carol
(86) Numéro de dépôt international: PCT/FR2000/001851
(87) Numéro de publication internationale: WO 2001/005454

(56) Documents cités:
- EP-A- 0 370 571
- DE-A- 19 607 922
- FR-A- 1 378 829
- US-A- 3 788 315
- US-A- 5 074 843

## Description

La présente invention est dans le domaine des seringues sans aiguille utilisées pour les injections intradermiques, sous-cutanées et intramusculaires, de principe actif liquide à usage thérapeutique en médecine humaine ou vétérinaire.

Dans ce domaine, pour augmenter l'efficacité de l'injection on utilise des seringues avec, à leur partie aval appliquée sur la peau ou très proche de la peau du sujet, un injecteur comportant plusieurs conduits afin de distribuer le liquide à injecter en plusieurs points répartis sur une surface relativement importante. Cette solution a aussi l'avantage de réduire la douleur et supprimer d'éventuelles altérations superficielles ou sous-cutanées dues à une trop grande quantité de liquide injecté en un seul point.

Pour augmenter l'efficacité de l'injection on joue aussi sur la forme du jet : on contrôle la distance de cohérence du jet et on recherche une solution intermédiaire entre un jet très cohérent, comme pour la découpe par jet qui pénétrerait très profondément et déchirerait dangereusement la peau et un jet qui nébulise le liquide et dont les fines gouttelettes ne pénètrent pas dans la peau.

Le brevet US 3 802 430 décrit une seringue sans aiguille dans laquelle le liquide à injecter est refoulé par un piston repoussé par des gaz produits par un générateur pyrotechnique ; cette seringue comporte cinq conduits parallèles à l'axe de la seringue et de sections transversales circulaires. Le brevet US 3 788 315 décrit une seringue dans laquelle le piston de refoulement du liquide est repoussé par la détente de gaz ou d'un ressort comprimés. Cette seringue comporte six conduits de sections transversales circulaires et dont les axes sont légèrement divergents de l'axe de la seringue. Dans ces exemples, bien que répartissant le liquide sur plusieurs points, les conduits restent assez proches les uns des autres ; de plus la simplicité de la forme de ces conduits montre que ces conduits ne sont pas optimisés pour contrôler la longueur de cohérence du jet qui elle, est un facteur important pour la performance de l'injecteur dans cette application particulière.

D'une façon plus générale les problèmes que pose la réalisation d'injecteur pour seringue sont des problèmes de résistance mécanique, de performances comme nous venons de l'évoquer et de coût.

En effet l'injecteur, placé à la partie aval de la seringue, ne doit pas se déformer sous l'effet de la pression du liquide lors de l'injection : l'injecteur doit être relativement épais, et cela d'autant plus que les conduits sont répartis sur une grande surface. Le problème va être de réaliser des conduits en général très fins sur de grandes épaisseurs.

La performance de l'injecteur réside dans la possibilité de contrôler la distance de cohérence des jets sortant des conduits ou buses, pour des conditions prédéterminées d'utilisation(nature du liquide, pression d'injection), par des conduits de sections transversales appropriées. Cette section transversale appropriée a pour but de créer un champ de turbulence dans l'écoulement tel que, à faible distance de la sortie de l'injecteur, le jet reste cohérent c'est-à-dire qu'il est assez fin et rapide pour percer et pénétrer dans la peau du sujet à traiter, ensuite le jet perd très rapidement de sa cohérence : il éclate pour diffuser au mieux le principe actif sous la peau. Le problème est de réaliser simplement, non seulement des conduits fins sur de grandes épaisseurs mais surtout des conduits avec des sections transversales appropriées.

Enfin le coût de fabrication devient un facteur très important lorsqu'il s'agit de seringues fabriquées en grande série notamment pour des seringues jetables.

La présente invention concerne une seringue sans aiguille pour l'injection intradermique, sous-cutanée ou intramusculaire d'un principe actif liquide initialement placé entre, d'une part un injecteur comportant au moins une buse d'injection, ledit injecteur étant placé au contact de la peau, où à très faible distance de la peau du sujet à traiter et d'autre part une paroi déplaçable, sous l'effet d'un système propulsif, assurant la mise en pression et l'expulsion du principe actif au travers de l'injecteur placé à l'extrémité aval de la seringue, qui est telle que, l'injecteur est constitué par au moins deux éléments dont les surfaces en contact sont dirigées vers la peau, au moins une des surfaces en contact comportant au moins une rainure qui constitue une buse d'injection dans l'assemblage des dits éléments.

Dans cette invention par principe actif liquide nous entendrons essentiellement un liquide plus ou moins visqueux, ou un mélange de liquides, ou un gel. Le principe actif pourra être un solide mis en solution dans un solvant approprié pour l'injection. Le principe actif pourra être un solide sous forme pulvérulente mis en suspension, plus ou moins concentrée, dans un liquide approprié. La granulométrie du principe actif solide doit être adaptée ainsi que la forme du conduit pour éviter les bouchages des conduits.

Un élément constitutif de l'injecteur comporte une face se situant du côté du principe actif : elle sera dite face amont, une face située du côté de la peau : elle sera dite face aval et une surface latérale dont tout ou partie est en contact avec tout ou partie de la surface latérale d'au moins un autre élément. Sur au moins une portion de surface latérale, au moins une rainure s'étend de la face amont à la face aval.

Dans l'assemblage des éléments constitutifs une rainure peut être en vis à vis de la surface latérale d'un élément adjacent ; elle peut être aussi en vis à vis d'une autre rainure tracée sur la face latérale de l'autre élément adjacent ; les rainures placées en vis à vis peuvent avoir des sections transversales identiques ou différentes.

Pour l'injecteur de la seringue dans une première configuration les surfaces de contact de deux éléments adjacents, surfaces de contact qui sont tout ou parties des surfaces latérales desdits éléments, sont des surfaces de révolution.

Dans une deuxième configuration d'injecteur les surfaces de contact de deux éléments adjacents, surfaces de contact qui sont tout ou parties des surfaces latérales desdits éléments, sont des surfaces planes.

Les dimensions transversales d'une rainure sont très petites vis à vis de la longueur de la rainure. On peut dire en première approximation que la rainure se positionne autour d'une courbe tracée sur une portion de surface latérale d'un élément constitutif de l'injecteur.

Dans une première réalisation cette courbe tracée sur la face latérale est sensiblement rectiligne : la rainure sera dite rectiligne. C'est par exemple le cas d'une rainure longitudinale faite sur une surface elle même plane, ou cylindrique ou conique.

Dans une deuxième réalisation cette courbe tracée sur la face latérale n'est pas rectiligne ou est une courbe gauche contenue dans aucun plan, la rainure sera dite hélicoïdale. C'est par exemple le cas d'une rainure hélicoïdale faite sur une surface cylindrique ou conique.

Dans une troisième réalisation cette rainure est formée par la convergence d'au moins deux portions de rainures débutant à partir de la face amont de l'élément et se terminant par une seule portion de rainure vers la face aval de l'élément. Les différentes portions de rainures sont rectilignes ou hélicoïdales.

Avantageusement la rainure a une section transversale sensiblement constante lorsqu'on suit la rainure de la face amont à la face aval de l'élément. Sa section transversale est, préférentiellement de forme géométrique simple, c'est par exemple une gorge en forme de « V », de « U », ou de demi-cercle. Ces formes présentent un plan de symétrie qui passe par un axe de symétrie de l'élément.

Préférentiellement la rainure a une section transversale évolutive. Lorsqu'on suit la rainure, depuis la face amont jusqu'à la face aval, sa section transversale varie ou évolue en augmentant ou diminuant, de façon régulière ou brusque, de sorte que la buse présente une succession de parties tubulaires et de cavités, la disposition convenable de ces différentes parties permettant de maîtriser et de contrôler la distance de cohérence du jet suivant des conditions prédéterminées d'utilisation : viscosité du liquide à injecter, pression d'injection notamment.

Une telle section transversale évolutive est par exemple réalisée simplement à partir d'une rainure de section transversale constante, comme celle précédemment décrite, sur laquelle on superpose au moins une empreinte qui élargit et approfondit localement la rainure. Dans l'assemblage des éléments de l'injecteur, les dites empreintes vont créer des cavités le long de la buse d'injection qui sera de section transversale évolutive, cette disposition permettant de contrôler la distance de cohérence du jet.

La convergence d'au moins deux rainures en une seule rainure peut aussi réaliser la section transversale évolutive tel qu'on l'entend ici.

Enfin la rainure peut être une succession d'empreintes très proches les unes des autres qui constituent la rainure à section transversale évolutive.

Dans une première réalisation de la seringue sans aiguille , l'injecteur comprend un support comportant au moins un logement dans lequel est emboîté un élément qui constitue un noyau monobloc. Lesdits noyau et support comportant des rainures pour réaliser au moins une buse d'injection.

Dans une deuxième réalisation de la seringue sans aiguille l'injecteur, comprend au moins un noyau constitué d'au moins deux éléments ou quartiers assemblés par leurs faces planes pour réaliser au moins une buse de section transversale évolutive, les éléments ou quartiers des différents noyaux étant emboîtés dans des logements d'un support.

Pour ces deux réalisations l'emboîtement est préférentiellement un emmanchement à force qui assure aussi l'étanchéité au niveau des surfaces de contact.

Le noyau monobloc ou constitué de plusieurs quartiers présente soit une symétrie de révolution ; le noyau a la forme d'un cylindre ou d'un tronc de cône soit une symétrie de répétition d'ordre n : le noyau a la forme d'un prisme ou d'un tronc de pyramide. Evidemment le logement qui reçoit le noyau a la même forme, il a une forme conjuguée pour faire l'emboîtement.

Dans un troisième mode de réalisation de la seringue sans aiguille l'injecteur comprend au moins un noyau constitué d'au moins deux quartiers assemblés par leurs faces planes pour réaliser au moins une buse à section transversale évolutive, les quartiers des différents noyaux étant solidarisés par un surmoulage.

Des assemblages gigognes, dans lesquels un sous ensemble comprenant un support ou surmoulage équipés de leurs noyaux fait aussi partie de l'invention. L'assemblage gigogne préféré est celui constitué par l'emboîtement d'un noyau dans un support comportant un seul logement, cet emboîtement servant de noyau pour un autre support à un seul logement. Une réalisation particulièrement simple consiste à emboîter un noyau directement dans l'extrémité aval de la seringue aménagée dans ce but.

Avantageusement pour des éléments de formes tronquées tels que des troncs de cônes, des troncs de pyramide ou des quartiers de tels éléments l'assemblage et le montage se fera de façon que la face aval soit celle de plus petite section : par ce montage la pression du liquide aura tendance à emboîter les éléments et non à les chasser de leur logement.

La présente invention concerne aussi un injecteur tel que le dit injecteur est constitué par au moins deux éléments dont les surfaces en contact sont dirigées vers la peau, au moins une des surfaces en contact comportant au moins une rainure qui constitue une buse d'injection dans l'assemblage des dits éléments.

Une seringue selon l'invention résout les problèmes posés. Pour la résistance de l'injecteur, l'augmentation de l'épaisseur ne présente pas de difficulté vis à vis de la réalisation de conduits fins avec des sections transversales évolutives ou non sur. de grandes épaisseurs.

Pour la performance de l'injecteur, l'invention permet de faire de façon simple, pour s'adapter aux conditions prédéterminés d'utilisation, le contrôle de la distance de cohérence des jets sortant des buses.

Pour l'aspect coût, l'injecteur comporte des éléments de formes simples et faciles à réaliser, par moulage direct des éléments de l'injecteur ou par usinage des rainures et des empreintes sur des ébauches fabriquées par ailleurs. L'ensemble de ces opérations de fabrications et d'assemblage se prête à une automatisation poussée.

La seringue selon l'invention présente de plus un avantage indéniable du point de vue de la sécurité en cas d'une utilisation anormale. Par exemple si la seringue est dirigée vers un visage et déclenchée accidentellement, les jets n'auront pas d'autres effets que d'arroser ledit visage de principe actif, sans aucun effet mécanique de percement, si la seringue n'est pas en contact (ou très proche) du visage. Cet avantage est lié à la maîtrise de la distance de cohérence du jet.

La présente invention va être décrite plus en détail à l'aide des figures suivantes.

La figure 1 représente, en coupe longitudinale partielle, une seringue selon l'invention.

La figure 2 représente, en coupe transversale, l'injecteur de ladite seringue.

Les figures 3 et 4 représentent, en perspective, des types de noyaux utilisables dans l'injecteur de la seringue précédemment représentée.

La figure 5 représente, vu en perspective, deux éléments d'un noyau selon un autre mode de réalisation de l'invention.

La figure 6 représente, en coupe longitudinale, un injecteur de seringue obtenu par l'assemblage d'éléments du type de ceux représentés sur la figure 5.

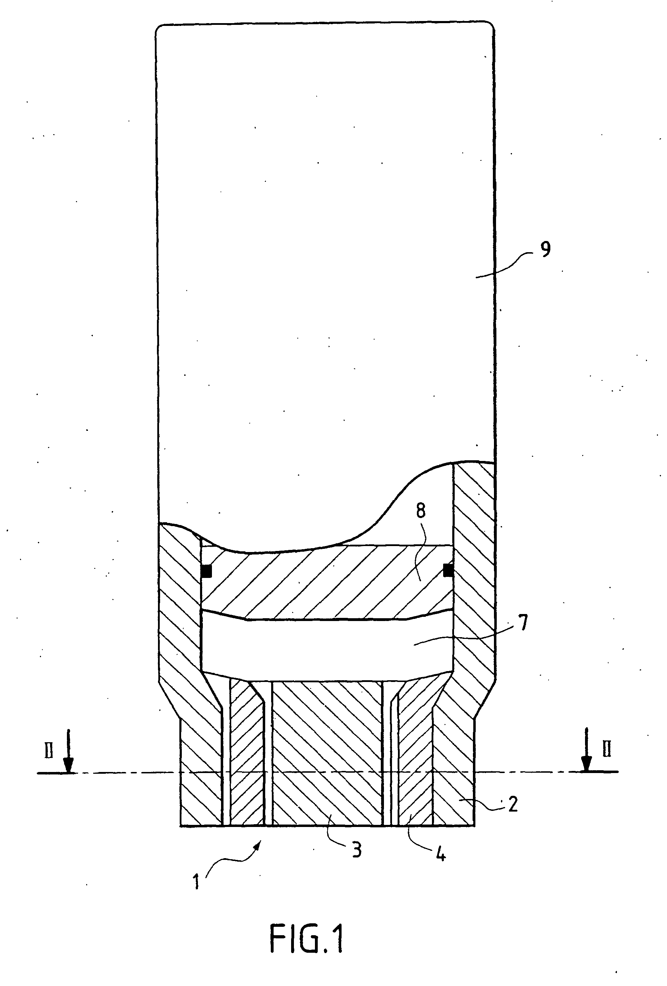

La figure 1 représente schématiquement une seringue sans aiguille pour l'injection de principe actif liquide. Une telle seringue est en général cylindrique et comporte un réservoir contenant le principe actif 7. Ce réservoir est fermé à une extrémité, que nous avons appelée extrémité aval 2, par un injecteur 1 comportant au moins un conduit ou une buse d'injection. Cet injecteur est en général en appui sur la peau du sujet à traiter, ou maintenue à très faible distance, la peau n'est pas représentée sur ce dessin. Cet injecteur est l'extrémité du réservoir, ou est une pièce rapportée 3, fixée sur cette extrémité du réservoir par des moyens appropriés. L'autre extrémité du réservoir est fermée par une paroi déplaçable, par exemple un piston 8 comportant des moyens pour assurer l'étanchéité tel qu'un joint torique. Enfin la seringue comporte un système propulsif 9 avec un dispositif de déclenchement pour déplacer le piston et injecter le liquide. Parmi les systèmes propulsifs utilisables et sans entrer dans leurs détails nous citerons un générateur pyrotechnique de gaz, comme décrit dans le brevet US 3 802 430 précédemment cité, nous citerons aussi la détente de gaz ou d'un ressort comprimés, comme décrit dans le brevet US 3 788 315. Il est évident que les seringues selon l'invention peuvent être équipées d'un quelconque de ces types de système propulsif pour déplacer le piston.

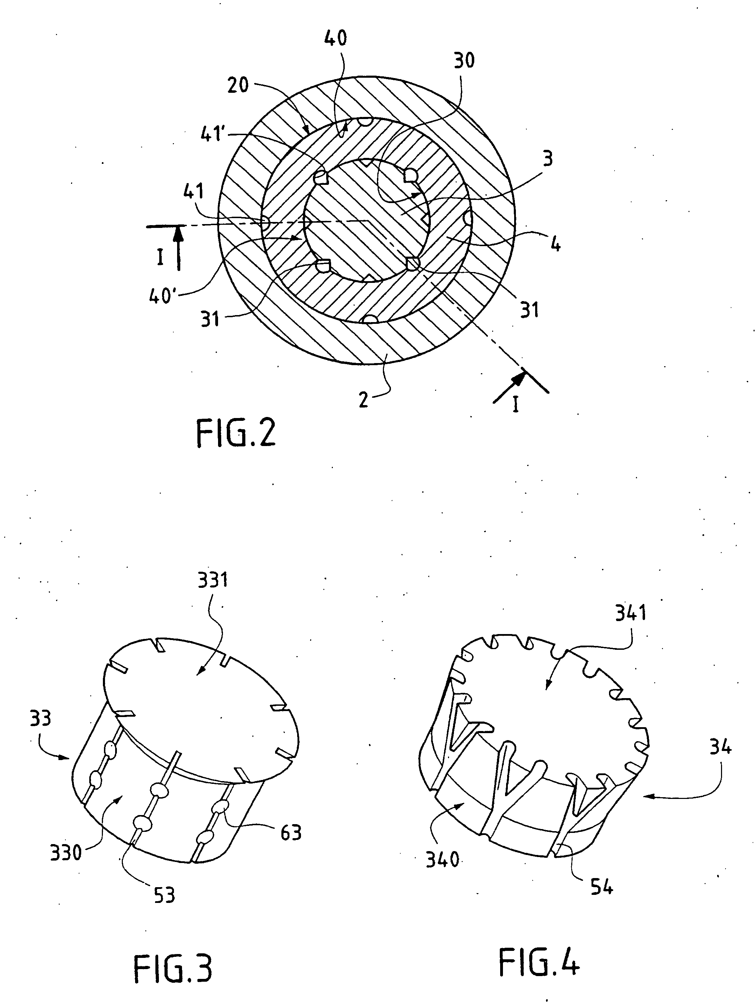

L'injecteur 1 (voir aussi fig. 2) est emmanché à force dans l'extrémité 2 de la seringue. Cet injecteur comporte un support 4 essentiellement cylindrique avec une face latérale extérieure 40 en appui sur la face latérale intérieure 20 de l'extrémité de la seringue et une face latérale intérieure 40' sur laquelle vient en contact la face latérale extérieure 30 d'un noyau 3, monobloc dans cet exemple. Le support 4 et le noyau 3 ont chacun, côté amont, un épaulement qui sert au blocage et au calage de ces éléments 3 et 4 dans l'assemblage ; cet épaulement est dans cet exemple tronconique. Les rainures sur les parois latérales extérieures se prolongent dans l'épaulement tronconique.

La figure 2 représente en coupe transversale l'extrémité aval 2 de la seringue précédemment décrite. Le support 4 est emboîté dans l'extrémité 2 de la seringue, les faces de contact étant respectivement les faces latérales 40 et 20'. Dans le support 4 est emboîté un noyau 3, les faces de contact étant respectivement les faces latérales 40' et 30. La face latérale extérieure 40 du support 4 comporte quatre rainures, telles que la rainure 41, elles sont équiréparties et ont sensiblement la forme d'un demi-cercle, les dites rainures sont en vis à vis de la surface latérale 20 de l'extrémité aval 2 de la seringue. La face latérale intérieure 40' du support 4 comporte aussi quatre rainures, telles que la rainure 41', semblables aux précédentes , aussi équiréparties, mais décalées de 45° par rapport aux rainures extérieures. Enfin la face latérale 30 du noyau 3 comporte huit rainures, telles que la rainure 31, elles sont équiréparties et ont une section transversale en forme de « V ». Parmi ces rainures, une sur deux, se trouve en vis à vis d'une rainure telle que la rainure 41', les autres rainures sont en vis à vis de la paroi latérale intérieure 40' du support.

Les rainures sur des parois latérales extérieures, rainures telles que les rainures 31 ou 41, se prolongent dans l'épaulement tronconique du noyau 3 et du support 4. Les rainures sur une face latérale intérieure telle que la rainure 41', sont dans le prolongement de l'ouverture dans l'épaulement de la rainure telle que 31 placée en vis à vis.

Les dimensions transversales des rainures sont telles qu'elles correspondent à des orifices circulaires de diamètre équivalent 0,05mm à 0,5mm. La hauteur d'un élément de l'injecteur est comprise entre environ 3mm et environ 10mm. Enfin les orifices des rainures sont réparties sur des cercles concentriques dont les diamètres sont compris entre 3mm et 30mm.

La figure 3 représente vu en perspective un noyau 33. Ce noyau se monte sur un support du type représenté sur les figures précédentes.

Le noyau 33 est essentiellement cylindrique circulaire, il comporte du côté amont un épaulement tronconique dont on voit la face amont 331. La surface latérale du noyau comporte huit rainures 53 équiréparties, elles sont longitudinales et de section semi-circulaire ; ces rainures se prolongent dans l'épaulement. Sur chaque rainure se trouvent deux empreintes 63 en forme de cône qui élargissent et approfondissent localement la rainure. Lorsque ce noyau sera emboîté dans un logement d'un support ces rainures et creux réalisent une buse de section transversale évolutive comportant dans cet exemple deux cavités qui vont générer des turbulences dans le jet et permettre ainsi de contrôler la distance de cohérence du jet.

Dans cet exemple le noyau a, sur la partie circulaire, un diamètre de 8mm et sa hauteur totale (avec épaulement) est de 5,8mm ; les rainures sont de forme semi-cylindrique dont le rayon est de 0,1mm, les cônes ont un angle au sommet de 90° et une base circulaire de 1mm de diamètre.

La figure 4 présente vu en perspective un autre noyau 34.
Ce noyau, à géométrie de révolution, est la combinaison d'une partie tronconique à l'amont et d'une partie cylindrique à l'aval, cette combinaison de forme réalise l'autoblocage du noyau dans son logement. La surface latérale 340 du noyau comporte 8 groupes de rainures équiréparties. La section transversale des rainures à la forme d'un « U ». Sur la partie tronconique de la surface latérale 340, depuis la face amont 341 deux portions de rainures identiques convergent pour se rassembler en une seule rainure, de même section transversale sur la partie cylindrique de la surface latérale pour déboucher sur la face aval. Dans cet exemple le cisaillement à la confluence de deux écoulements va générer la turbulence pour contrôler la distance de cohérence du jet.

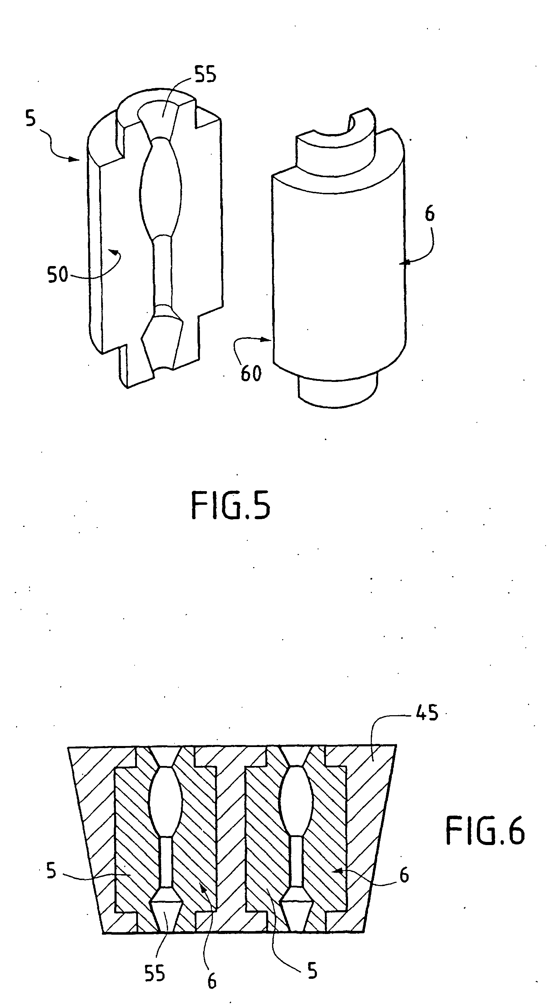

La figure 5 représente en perspective un noyau constitué de plusieurs parties ou quartiers, dans cet exemple deux parties 5 et 6 ayant essentiellement la forme de deux demi-cylindres représentés nettement séparés pour le lisibilité du schéma. Ces deux demi-cylindres 5 et 6 seront accolées par leurs faces planes 50 et 60 (cette dernière est cachée pour l'élément 6). Dans cet exemple chaque face plane comporte en son milieu, une rainure à section transversale évolutive. Compte-tenu de son aspect un peu différent de ce qui a été précédemment décrit nous l'appellerons aussi empreinte, mais ladite empreinte est bien une rainure à section transversale évolutive selon l'invention.

La figure 6 représente, en coupe transversale un injecteur 10 constitué de deux noyaux comprenant deux pièces 5,6 assemblées dans un surmoulage 45 ; les pièces 5,6, ont à leurs deux extrémités une portion de diamètre réduit qui réalisent des épaulements pour le centrage des éléments dans le surmoulage. Dans l'assemblage des quartiers tels que 5 et 6 les empreintes en vis à vis forment un conduit 55 à symétrie de révolution et de section transversale évolutive comprenant, dans cet exemple, depuis la face amont jusqu'à la face aval, un tronc de cône se raccordant à une cavité oblongue, puis une portion cylindrique circulaire se raccordant à une cavité formée de deux troncs de cône inégaux raccordés par leurs bases la plus large.

D'une façon générale l'épaisseur d'un élément de l'injecteur, distance de la face amont à la face aval, est comprise entre environ 3mm et environ 10mm. Les dimensions des sections transversales des rainures ou des empreintes évoluent et sont telles que l'aire correspond à celle d'un conduit circulaire de diamètre varie entre 50 µm environ et 1000µm.

Les matériaux pour réaliser la seringue et les différentes parties de la buse seront choisis parmi les matériaux compatibles et agrées pour un usage médical ; sans prétendre être exhaustif nous citerons par exemple des matériaux plastiques tels que les polycarbonate, des polytétrafluors éthylènes ; des métaux ; inox, ou du verre à usage médical de type I ou II.

## Revendications

1. Seringue sans aiguille pour l'injection d'un principe actif (7) initialement placé entre, d'une part un injecteur (1,10) comportant au moins une buse d'injection, ledit injecteur étant placé à l'extrémité aval (2) de la seringue, et d'autre part une paroi déplaçable (8) sous l'effet d'un système propulsif (9) assurant la mise en pression et l'expulsion du principe actif au travers de l'injecteur, **caractérisée en ce que**, l'injecteur (1,10) est constitué par l'assemblage d'au moins deux éléments (3,4,5,6,33,34) ; chaque élément ayant une face aval, une face amont et une surface latérale les reliant, les surfaces en contact (30, 40, 40', 50, 60, 330, 340) desdits éléments dans l'assemblage étant tout ou partie des surfaces latérales desdits éléments ; au moins une des surfaces en contact comportant au moins une rainure (31,41,41',53,54,55) qui constitue une buse d'injection dans l'assemblage des dits éléments.

2. Seringue sans aiguille selon la revendication 1 **caractérisée en ce que** les surfaces en contact (30,40,40') sont des surfaces de révolution.

3. Seringue sans aiguille selon la revendication 1 **caractérisée en ce que** les surfaces de contact (50,60) sont des surfaces planes.

4. Seringue sans aiguille selon l'une des revendications 1,2 ou 3 **caractérisée en ce que** la rainure (31,41,41') est rectiligne.

5. Seringue sans aiguille selon l'une des revendications 1 ou 2 **caractérisée en ce que** la rainure est hélicoïdale.

6. Seringue sans aiguille selon l'une des revendications 1,2 ou 3 **caractérisée en ce qu'**une rainure (54) est formée par la convergence d'au moins deux rainures, débutant à partir de la face amont, se terminant par une seule rainure vers la face aval de l'élément (34).

7. Seringue selon l'une des revendications 4,5 ou 6 **caractérisée en ce que** la rainure (31,41,41')a une section transversale constante.

8. Seringue selon l'une des revendications 4,5 ou 6 **caractérisée en ce que** la rainure (53,54,55) a une section transversale évolutive.

9. Seringue sans aiguille selon l'une des revendications 1,2,3 ou 8 **caractérisée en ce que** l'injecteur (1) comprend un support (4) comportant un logement dans lequel est emboîté un noyau monobloc (3,33,34).

10. Seringue sans aiguille selon l'une des revendications 1,3 ou 8 **caractérisée en ce que** l'injecteur, comprend au moins un noyau constitué d'au moins deux quartiers assemblés par leurs faces planes pour réaliser au moins un noyau avec une buse de section transversale évolutive, les quartiers des différents noyaux étant emboîtés dans des logements d'un support.

11. Seringue sans aiguille selon l'une des revendications 1,2,3 ou 8 **caractérisée en ce que** l'injecteur (10) comprend au moins un noyau constitué d'au moins deux quartiers (5,6,) assemblés par leurs faces planes (50,60) pour réaliser au moins un noyau avec une buse (55) à section transversale évolutive, les quartiers (5,6,) des différents noyaux étant solidarisés par un surmoulage (45).

12. Injecteur (1,10), pour seringue sans aiguille, **caractérisé en ce que** le dit injecteur est constitué par au moins deux éléments (3,4,5,6,30,33,34) ; chaque élément ayant une face aval parallèle à la face aval de l'injecteur, une face amont et une surface latérale les reliant, les surfaces en contact (30, 40, 40', 50, 60, 330, 340) desdits éléments dans l'assemblage étant tout ou partie des surfaces latérales desdits éléments ; au moins une des surfaces en contact comportant au moins une rainure (31,41,41',53,54,55) qui constitue une buse d'injection dans l'assemblage des dits éléments.

## Patentansprüche

1. Nadellose Spritze zum Spritzen eines Wirkstoffs (7), der ursprünglich zwischen einerseits einem Injektor (1, 10), der mindestens eine Einspritzdüse aufweist, wobei der Injektor am stromabwärts liegenden Ende (2) der Spritze angeordnet ist, und andererseits einer unter der Wirkung eines Antriebssystems (9) verschiebbaren Wand (8) angeordnet ist, die das Unterdrucksetzen und den Ausstoß des Wirkstoffs durch den Injektor hindurch gewährleistet, **dadurch gekennzeichnet, dass** der Injektor (1, 10) aus dem Zusammenbau von mindestens zwei Elementen (3, 4, 5, 6, 33, 34) besteht, wobei jedes Element eine stromabwärts liegende Seite, eine stromaufwärts liegende Seite, und eine diese Seiten verbindende Seitenfläche aufweist, wobei die in Kontakt stehenden Flächen (30, 40, 40', 50, 60, 330, 340) dieser Elemente in dem Zusammenbau ganz oder zum Teil Seitenflächen der Elemente sind, wobei mindestens eine der in Kontakt stehenden Flächen mindestens eine Nut (31, 41, 41', 53, 54, 55) aufweist, die im Zusammenbau der Elemente eine Einspritzdüse bildet.

2. Nadellose Spritze nach Anspruch 1, **dadurch gekennzeichnet, dass** die in Kontakt stehenden Flächen (30, 40, 40') drehsymmetrische Flächen sind.

3. Nadellose Spritze nach Anspruch 1, **dadurch gekennzeichnet, dass** die in Kontakt stehenden Flächen (50, 60) ebene Flächen sind.

4. Nadellose Spritze nach einem der Ansprüche 1, 2 oder 3, **dadurch gekennzeichnet, dass** die Nut (31, 41, 41') geradlinig ist.

5. Nadellose Spritze nach einem der Ansprüche 1 oder 2, **dadurch gekennzeichnet, dass** die Nut schraubenförmig ist.

6. Nadellose Spritze nach einem der Ansprüche 1, 2 oder 3, **dadurch gekennzeichnet, dass** eine Nut (54) von der Konvergenz mindestens zweier Nuten gebildet wird, die an der stromaufwärts liegenden Seite beginnen und zur stromabwärts liegenden Seite des Elements (34) hin in einer einzigen Nut enden.

7. Spritze nach einem der Ansprüche 4, 5 oder 6, **dadurch gekennzeichnet, dass** die Nut (31, 41, 41') einen konstanten Querschnitt aufweist.

8. Spritze nach einem der Ansprüche 4, 5 oder 6, **dadurch gekennzeichnet, dass** die Nut (53, 54, 55) einen fortschreitenden Querschnitt aufweist.

9. Nadellose Spritze nach einem der Ansprüche 1, 2, 3 oder 8, **dadurch gekennzeichnet, dass** der Injektor (1) einen Träger (4) aufweist, der einen Sitz enthält, in den ein einstückiger Kern (3, 33, 34) eingepasst ist.

10. Nadellose Spritze nach einem der Ansprüche 1, 3 oder 8, **dadurch gekennzeichnet, dass** der Injektor mindestens einen Kern aufweist, der aus mindestens zwei Stücken besteht, die mit ihren ebenen Seiten zusammengesetzt werden, um mindestens einen Kern mit einer Düse mit fortschreitendem Querschnitt zu bilden, wobei die Stücke der verschiedenen Kerne in Sitze eines Trägers eingepasst sind.

11. Nadellose Spritze nach einem der Ansprüche 1, 2, 3 oder 8, **dadurch gekennzeichnet, dass** der Injektor (10) mindestens einen Kern aufweist, der aus mindestens zwei Stücken (5, 6) besteht, die mit ihren ebenen Seiten (50, 60) zusammengesetzt werden, um mindestens einen Kern mit einer Düse (55) mit fortschreitendem Querschnitt herzustellen, wobei die Stücke (5, 6) der verschiedenen Kerne durch einen Überguss (45) aneinander befestigt werden.

12. Injektor (1, 10) für eine nadellose Spritze, **dadurch gekennzeichnet, dass** der Injektor aus mindestens zwei Elementen (3, 4, 5, 6, 30, 33, 34) besteht, wobei jedes Element eine stromabwärts liegende Seite parallel zur stromabwärts liegenden Seite des Injektors, eine stromaufwärts liegende Seite und eine diese Seiten verbindende Seitenfläche hat, wobei die in Kontakt stehenden Flächen (30, 40, 40', 50, 60, 330, 340) der Elemente im Zusammenbau ganz oder zum Teil Seitenflächen der Elemente sind, wobei mindestens eine der in Kontakt stehenden Flächen mindestens eine Nut (31, 41, 41', 53, 54, 55) aufweist, die im Zusammenbau der Elemente eine Einspritzdüse bildet.

## Claims

1. Needleless syringe for injecting an active principle (7) initially placed between, on the one hand, an injector (1,10) having at least one injection nozzle, the said injector being placed at the downstream end (2) of the syringe and, on the other hand, a wall (8) that can be displaced under the effect of a propellant system (9) ensuring that the active principle is put under pressure and expelled through the injector, **characterized in that** the injector (1,10) consists of the assembly of at least two elements (3,4,5,6,33,34); each element having a downstream face, an upstream face and a lateral face connecting them, the contacting surfaces (30, 40, 50', 50, 60, 330, 340) of the said elements in the assembly being all or part of the lateral surfaces of the said elements; at least one of the contacting surfaces having at least one groove, (31, 41, 41' 53, 54, 55) which constitutes an injection nozzle in the assembly of the said elements.

2. Needleless syringe according to claim 1 **characterized in that** the contacting surfaces (30,40,40') are surfaces of revolution.

3. Needleless syringe according to claim 1, **characterized in that** the contacting surfaces (50,60) are plane surfaces.

4. Needleless syringe according to one of claims 1,2 or 3 **characterised in that** the groove (31,41,41') is rectilinear.

5. Needleless syringe according to one of claims 1 or 2 **characterized in that** the groove is helical.

6. Needleless syringe according to one of claims 1,2 or 3 **characterized in that** a groove (54) is formed by the convergence of at least two grooves, starting from the upstream face and terminating by a single groove towards 5 the downstream face of the element (34).

7. Syringe according to one of claims 4,5 or 6, **characterized in that** the groove (31,41,41') has a constant transverse section.

8. Syringe according to one of claims 4,5 or 6 **characterized in that** the groove (53,54,55) has a progressively changing transverse section.

9. Needleless syringe according to one of claims 1,2,3 or 8 **characterized in that** the injector (1) includes a support (4) having a housing into which a single-piece core (3,33,34) is fitted.

10. Needleless syringe according to one of claims 1,3 or 8 **characterized in that** the injector includes at least one core consisting of at least two quarters assembled by their plane faces to produce at least one core with a nozzle having a progressively changing transverse section, the quarters of the different cores being fitted into housings in a support.

11. Needleless syringe according to one of claims 1,2,3 or 8 **characterized in that** the injector (10) includes at least one core consisting of at least two quarters (5,6) assembled by their plane faces (50,60) to produce at least one core having a nozzle (55) with a progressively changing transverse section, quarters (5,6) of the different cores being secured by an overmoulding (45).

12. Injector (1,10) for a needleless syringe, **characterized in that** the said injector consists of at least two elements (3,4,5,6,30,33,34); each element having a downstream face parallel to the downstream face of the injector, an upstream face and a lateral surface connecting them, the contacting surfaces (30,40,40'50,60,330,340) of the said elements in the assembly being all or part of the lateral surfaces of the said elements; at least one of the contacting surfaces having at least one groove (31,41,41'53,54,55) which constitutes an injection nozzle in the assembly of the said elements.
